# EUROPEAN PATENT APPLICATION

(11) **EP 3 009 062 A1**
(43) Date of publication of application: **20.04.2016**
(21) Application number: 14810360.9
(22) Date of filing: 07.01.2014
(51) Int. Cl.: A61B 1/00

(54) **ENDOSCOPE**

(30) Priority: 11.06.2013 JP 2013122840
(71) Applicant: OLYMPUS CORPORATION, Shibuya-ku Tokyo 151-0072 (JP)
(72) Inventor: KUMAGAI, Kazutoshi, Tokyo 151-0072 (JP); SUGIMOTO, Naoya, Tokyo 151-0072 (JP); UEKI, Kei, Tokyo 151-0072 (JP); OKAZAKI, Yoshiro, Tokyo 151-0072 (JP)
(74) Representative: Gunzelmann, Rainer
(86) International application number: PCT/JP2014/050055
(87) International publication number: WO 2014/199648

(57) **Abstract**

Provided is an endoscope (1A) including an inserted portion (2) that is inserted into a body, an observation optical system (3) that is provided at a distal-end portion of the inserted portion (2) and that acquires an image of the heart, and a supporting expansion portion (4A) that makes the heart support the distal-end portion of the inserted portion (2) and that is provided with a basal-end-portion balloon (8), which is provided closer to a base-end portion than the distal-end portion of the inserted portion (2) is, and a distal-end-portion balloon (7), which is provided so as to overhang forward from the distal-end portion of the inserted portion (2), wherein the base-end-portion balloon (8) is disposed on the opposite side from the distal-end-portion balloon (7), with the inserted portion (2) interposed therebetween.

## Description

### {Technical Field}

The present invention relates to an endoscope.

### {Background Art}

There are known techniques in which an endoscope is inserted into the pericardial cavity between the heart and the parietal pericardium to directly diagnose a diseased portion or to treat a diseased portion while viewing the diseased portion (for example, see Patent Literatures 1 and 2).

When an endoscope is employed in the case in which the heart is the treatment subject, there is a problem in that the distal-end portion of the endoscope is uncontrollably moved in the pericardial cavity due to the influence of the beating heart, thus making it impossible to ensure a stable viewing field.

As a solution, Patent Literature 1 discloses an endoscope that is provided with, at least at the distal-end portion of an inserted portion that is inserted into the body of the patient, a securing means for securing the inserted portion to tissue inside the body and an observation-distance adjusting means for adjusting the distance between an observation optical system provided in the inserted portion and the tissue surface.

With this endoscope, by inserting the inserted portion into the body of the patient and by securing at least the distal-end portion of the inserted portion to the tissue inside the body by using the securing means, regardless of the presence/absence of pulsation or the like of the tissue, it is possible to ensure a stable viewing field by allowing the observation optical system provided in the inserted portion to follow the pulsation of the tissue. Then, by adjusting the distance between the observation optical system and the tissue surface by operating the observation-distance adjusting means, it is possible to observe the tissue surface with the observation optical system from an appropriate distance.

In addition, Patent Literature 2 discloses an endoscope that is provided with an inserted portion that is inserted into the body, a bending portion that is bendable so as to change the direction in which the distal-end surface of the inserted portion faces, and a protruded portion that protrudes further forward than the distal-end surface.

With this endoscope, when the inserted portion is inserted along the heart surface and the bending portion is bent so that the distal-end surface faces the heart surface, the furthest distal-end portion of the protruded portion is temporarily secured to the heart surface, and thus, the rotation of the endoscope in the circumferential direction with respect to the heart surface is restricted. In other words, even if the heart is beating, the distal-end portion of the endoscope can be placed in a stable orientation with respect to the heart surface and diagnosis or treatment can easily be performed at a desired position

### {Citation List}

### {Patent Literature}

{PTL 1} Japanese Unexamined Patent Application, Publication No. 2010-284503
{PTL 2} Japanese Unexamined Patent Application, Publication No. 2010-207455

### {Summary of Invention}

### {Technical Problem}

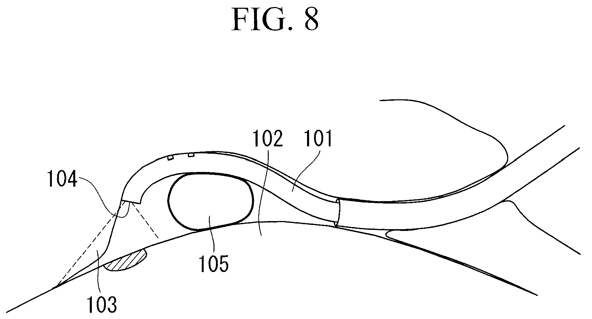

As shown in Fig. 8, with the configuration described in Patent Literature 1, an endoscope 101 is inserted between an epicardium 102 and a parietal pericardium 103, a balloon 105 that serves as an observation-distance adjusting means is inflated, and observation is performed by using an observation optical system 104. At this time, because the parietal pericardium 103 hangs over in front of the observation optical system 104 provided at the distal-end portion of the endoscope 101, the viewing field of the observation optical system 104 may be narrowed or it may not be possible to ensure a sufficient space for performing treatment.

With the configuration of Patent Literature 2, because the protruded portion abuts the heart, it is possible to prevent adverse effects on the viewing field by means for the protruded portion even if the parietal pericardium hangs over. However, because the distance between the heart and the observation optical system is extremely small in this configuration, the observation viewing field ends up being narrowed.

With a narrow observation viewing field, it is difficult to judge which part of the heart is being observed. Therefore, when determining a portion to be treated, the portion requiring treatment is determined by observing a large area first, and subsequently, the portion requiring treatment is observed in detail. Furthermore, after treating the subject portion while performing localized detailed observation, evaluation must be performed over a large area that includes the subject portion and the surrounding area thereof. However, when it is not possible to ensure a sufficient observation viewing field, it is difficult to perform diagnosis or treatment because a large area cannot be observed.

The present invention has been conceived in light of the above-described circumstances, and an object thereof is to provide an endoscope with which it is possible to prevent narrowing of an observation viewing field and with which it is also possible to ensure a sufficient space for performing treatment.

### {Solution to Problem}

An aspect of the present invention is an endoscope including an inserted portion that is inserted into a body; an observation optical system that is provided at a distal-end portion of the inserted portion and that acquires an image of the body interior; and a supporting expansion portion that makes an organ inside the body support the distal-end portion of the inserted portion and that is provided with a first expanding member, which is provided closer to a base-end portion than the distal-end portion of the inserted portion is, and a second expanding member, which is provided so as to overhang forward from the distal-end portion of the inserted portion, wherein, when viewing a cross-section perpendicular to a center axis of the inserted portion, the first expanding member is disposed on the opposite side from the second expanding member, with the inserted portion interposed therebetween.

With this aspect, by making the first expanding member face the organ such as the heart or the like, which serves as an observation/treatment subject, the inserted portion is moved away from the surface of the organ. By doing so, a sufficient distance is ensured between the observation optical system and the surface of the organ, and thus, an appropriate observation distance is formed therebetween. In addition, the second expanding member is made to face the other membranes and organs that are positioned in the area surrounding the organ such as the heart or the like, which is the observation/treatment subject. With this second expanding member overhanging forward from the distal-end portion of the inserted portion, the second expanding member extends forward from the distal-end portion of the inserted portion similarly to eaves, supports the other membranes and organs that are positioned in the area surrounding the organ, which is the observation/treatment subject, and prevents the other membranes and organs from sagging. As a result, it is possible to ensure a sufficient space for performing treatment by expanding the space in front of the viewing field of the observation optical system.

In the above-described aspect, the first expanding member may be a first balloon that is inflated toward an outer circumferential side from the inserted portion, and the second expanding member is a second balloon that is inflated toward the outer circumferential side from the inserted portion and that is inflated so as to overhang further forward than the distal-end portion of the inserted portion.

By doing so, when the first balloon that serves as the first expanding member is inflated, the inserted portion is moved away from the surface of the organ, such as the heat or the like, in the body. In addition, when the second balloon that serves as the second expanding member is inflated, the other membranes and organs that are positioned in the area surrounding the organ, which is the observation/treatment subject, are supported.

In the above-described aspect, the supporting expansion portion may be additionally provided with a third balloon provided closer to the base-end portion of the inserted portion than the first balloon is and that bends the inserted portion, and the third balloon may be disposed on the opposite side from the first balloon, with the inserted portion interposed therebetween.

When the third balloon is inflated, the outer surface of the third balloon comes into contact with other membranes, organs, or the like, and the third balloon presses the inserted portion toward the organ by receiving a reaction force from the other membranes, organs, or the like. By doing so, the inserted portion is bent.

In the above-described aspect, in a state in which the first balloon is inflated and the inserted portion is moved away from a surface of the organ, the third balloon may be provided between a portion at which the first balloon is in contact with the inserted portion and a portion at which the inserted portion is in contact with the surface of the organ.

By doing so, the third balloon presses the inserted portion toward the organ between the first portion at which the first balloon is in contact with the inserted portion and the second portion that is closer to the base end of the inserted portion than the first portion is and at which the inserted portion is in contact or in the close vicinity of the surface of the organ. As a result, the inserted portion is bent and is raised in the direction that moves the inserted portion away from the outer circumferential side of the organ from the position aligned with the organ, after which the distal-end portion is aligned with the second balloon by the other membranes and organs. By doing so, the distal-end portion of the inserted portion is made nearly parallel to the surface of the organ and the other membranes, organs, or the like. By doing so, it is possible to perform observation and treatment by placing the inserted portion parallel to the surface of the organ.

In the above-described aspect, the supporting expansion portion may be formed by being provided with a material that blocks an X-ray.

By doing so, it is possible to easily ascertain positions of and degrees-of-inflation of the individual balloons inside the body by radiating X-rays from outside the body.

In the above-described aspect, an outer surface of the first balloon that is on an opposite side from a side that faces the inserted portion may serve as a concave curved surface that forms a concavity on an opposite side from the inserted portion when viewed from a center of the first balloon.

By doing so, the first balloon conforms to the surface of the organ, and thus, it is possible to stably support the inserted portion by preventing the inserted portion from rotating about the axis thereof.

In the above-described aspect, an outer surface of the second balloon on an opposite side from a side that faces an outer circumferential surface of the inserted portion may serve as a convex curved surface that forms a convexity on an opposite side from the inserted portion when viewed from a center of the second balloon.

By doing so, the second balloon conforms to other membranes, organs, or the like, and thus, it is becomes easier to stabilize the orientation of the inserted portion with respect to the other membranes, organs, or the like.

In the above-described aspect, the first balloon may be provided only at a portion of an outer circumferential surface of the inserted portion in a circumferential direction, and the outer circumferential surface of the inserted portion may be exposed at a remaining portion in the circumferential direction.

By doing so, as compared with the case in which the first balloon is provided so as to be continuous over the entire circumference of the inserted portion in the circumferential direction, it is possible to move the inserted portion further away from the organ. Accordingly, the viewing field of the observation optical system can be made larger.

In the above-described aspect, the first balloon may be provided closer to the base-end portion than the bending portion of the inserted portion is.

By doing so, the first balloon is prevented from hindering the bending operation of the bending portion.

In the above-described aspect, at least one of the first balloon and the second balloon may have, at a cross-section perpendicular to the center axis of the inserted portion, a width set to be larger than an outer diameter of the inserted portion.

By doing so, stability is increased because the surface areas of the first balloon and the second balloon that are in contact with organs and membranes are increased, and it is possible to prevent the inserted portion from rotating about the center axis thereof.

In the above-described aspect, at least the width of the first balloon may be set to be larger than the outer diameter of the inserted portion.

As a means for suppressing the rotation of the inserted portion, it is more effective to increase the width of the first balloon that is on the side that faces the organ, which is the observation/treatment subject.

In the above-described aspect, an outer surface of the third balloon on an opposite side from a side that faces the inserted portion may serve as a convex curved surface that forms a convexity on an opposite side from the inserted portion when viewed from a center of the third balloon.

By dong so, the outer surface of the third balloon conforms to the other membranes and organs, which facilitates stabilization thereof.

In the above-described aspect, the third balloon may be provided only at a portion of the inserted portion in the circumferential direction, and the outer circumferential surface of the inserted portion may be exposed at a remaining portion in the circumferential direction.

In the above-described aspect, at least one of the first balloon, the second balloon, and the third balloon may have, at a cross-section perpendicular to the center axis of the inserted portion, a width set to be larger than an outer diameter of the inserted portion.

By doing so, because surface areas that are in contact with organs and membranes are increased, it is possible to stably support the inserted portion by preventing the inserted portion from rotating about the center axis thereof.

### {Advantageous Effects of Invention}

The present invention affords an advantage in that it is possible to prevent narrowing of an observation viewing field and that it is also possible to ensure a sufficient space for performing treatment.

### {Brief Description of Drawings}

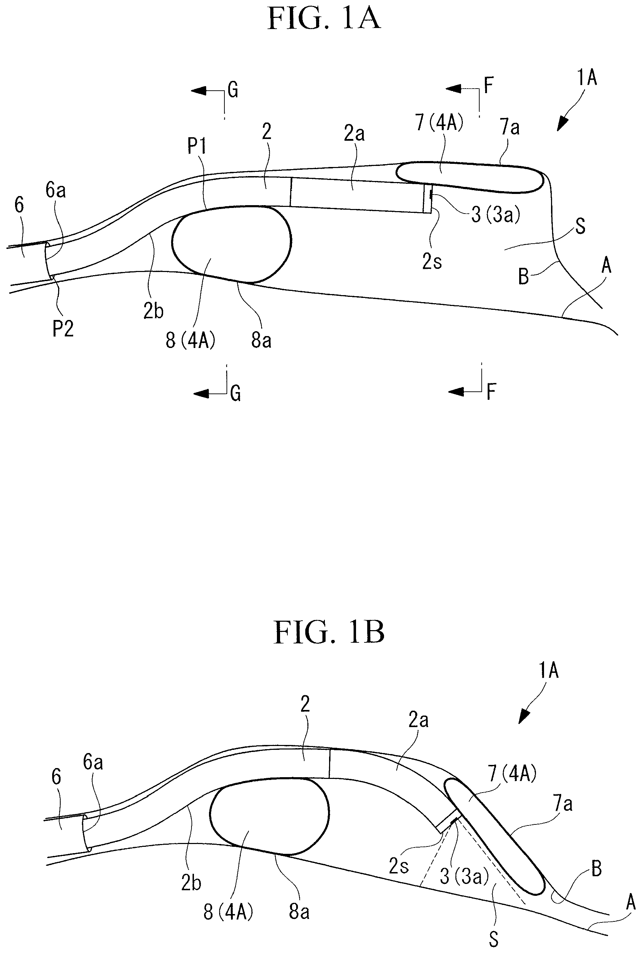

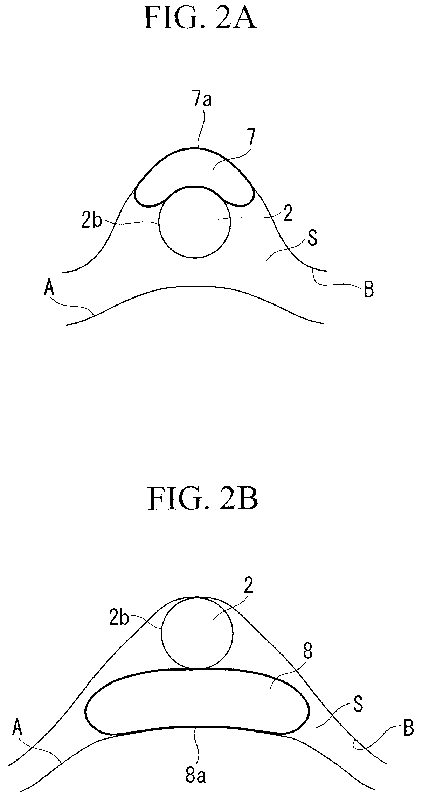

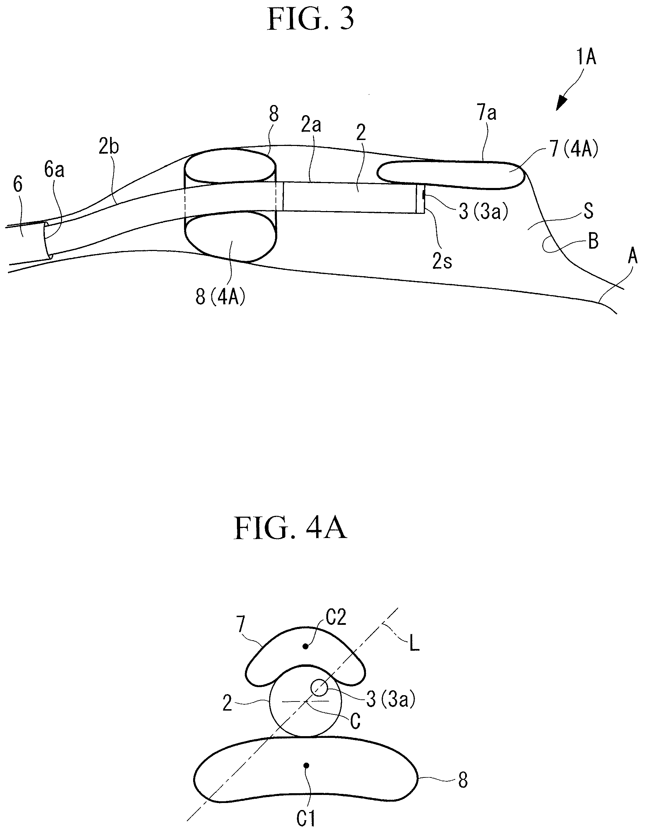

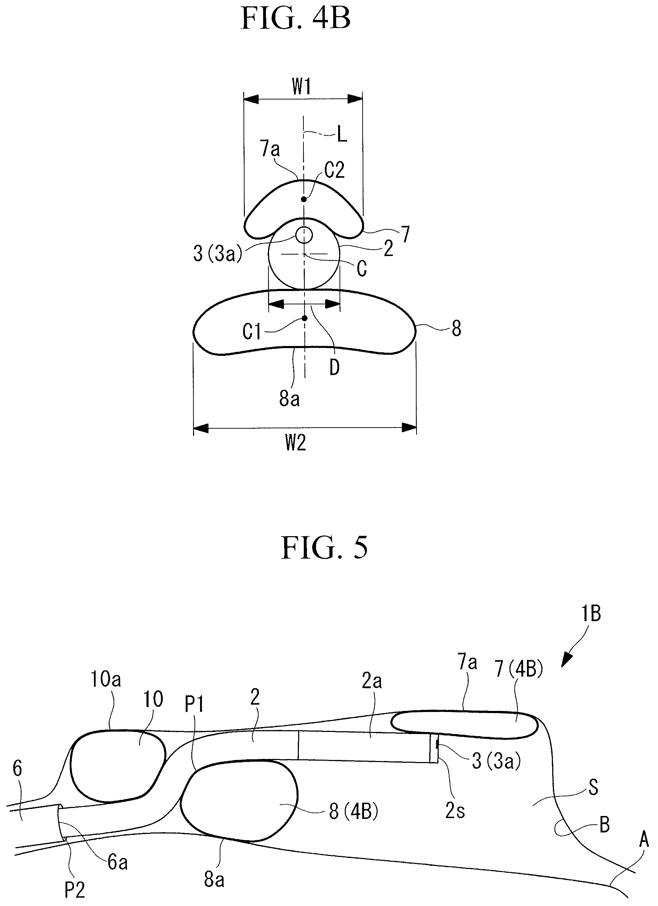

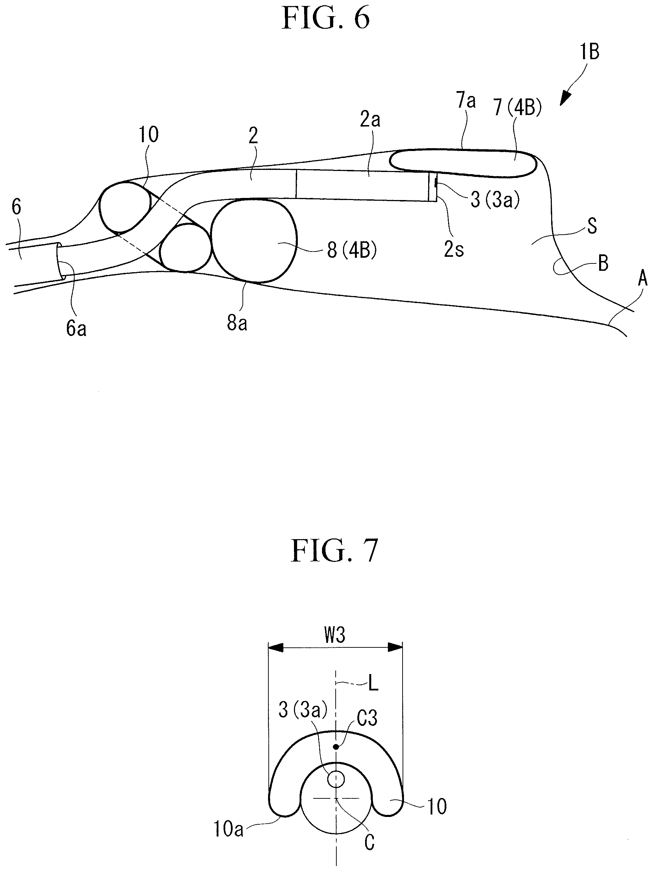

{Fig. 1A} Fig. 1A is a diagram showing a state in which a bending portion of an inserted portion of an endoscope according to a first embodiment of the present invention is kept straight.
{Fig. 1B} Fig. 1B is a diagram showing a state in which the bending portion of the inserted portion of the endoscope according to the first embodiment of the present invention is bent.
{Fig. 2A} Fig. 2A is a diagram showing a sectional view of the endoscope according to the first embodiment of the present invention taken along F-F in Fig. 1A.
{Fig. 2B} Fig. 2B is a diagram showing a sectional view of the endoscope according to the first embodiment of the present invention taken along G-G in Fig. 1A.
{Fig. 3} Fig. 3 is a diagram showing a modification of the endoscope according to the first embodiment of the present invention.
{Fig. 4A} Fig. 4A is a sectional view of the inserted portion in the short-axis direction, showing an example positional relationship between a first balloon and a second balloon in the endoscope according to the first embodiment of the present invention.
{Fig. 4B} Fig. 4B is a diagram showing a modification of the endoscope according to the first embodiment of the present invention in which a center C1 of a base-end-portion balloon 8 and a center C2 of a distal-end-portion balloon 7 exist on a virtual line L.
{Fig. 5} Fig. 5 is a diagram showing the configuration of a relevant portion of an endoscope according to a second embodiment of the present invention.
{Fig. 6} Fig. 6 is a diagram showing a modification of the endoscope according to the second embodiment of the present invention.
{Fig. 7} Fig. 7 is a sectional view of the endoscope according to the second embodiment of the present invention.
{Fig. 8} Fig. 8 is a diagram showing an example endoscope according to the Background Art.

### {Description of Embodiments}

Endoscopes according to embodiments of the present invention will be described below with reference to the drawings.

### {First Embodiment}

As shown in Figs. 1A and 1B, an endoscope 1A according to a first embodiment of the present invention is provided with a long, thin, flexible inserted portion 2 that is inserted into the body, an observation optical system 3 that is provided at a distal-end surface 2s of the inserted portion 2, in which a center axis C thereof extends in a direction nearly perpendicular to the distal-end surface 2s, and that acquires an image in over a wide angle in the forward direction, and a supporting expansion portion 4A that expands a space in front of the viewing field of the observation optical system 3 while making an organ (for example, a heart A) inside the body support the distal-end portion of the inserted portion 2.

The inserted portion 2 is inserted into the body cavity through a cylindrical guide sheath 6. The guide sheath 6 possesses flexibility, which makes the guide sheath 6 bendable in accordance with the tissue shape in the body cavity, and has an inner diameter that allows the inserted portion 2 and other medical devices to be inserted thereinto.

Figs. 1A and 1B are diagrams showing states of the distal end of the inserted portion 2 of the endoscope 1A that is inserted into a pericardial cavity S between the heart A and a parietal pericardium B via the guide sheath 6. The inserted portion 2 has a smaller outer diameter than the inner diameter of the guide sheath 6 and is configured so as to be inserted into the pericardial cavity S in a state in which the inserted portion 2 is accommodated inside the guide sheath 6 so as to be protrudable from and retractable into a distal-end opening 6a of the guide sheath 6.

The inserted portion 2 is provided with, at the distal-end portion thereof, a bending portion 2a that is bent in order to make the distal-end surface 2s face an arbitrary direction. Therefore, the term distal-end portion as used in this embodiment can be redefined as a portion of the inserted portion 2 that is closer to the distal end thereof than the bending portion 2a is, including the bending portion 2a and the distal-end surface 2s.

An observation window 3a that is arranged at the distal-end surface 2s of the inserted portion 2, an objective lens (not shown) that is provided in the inserted portion 2 and that collects light entering via the observation window 3a from over a wide angle in the forward direction where the center axis C extends in the direction perpendicular to the distal-end surface 2s of the inserted portion 2, and an image-acquisition device (not shown), such as a CCD, for imaging the light collected by the objective lens are disposed in the observation optical system 3.

The supporting expansion portion 4A is provided with a distal-end-portion balloon (second expanding member, second balloon) 7 provided at an external side surface of the distal-end portion of the inserted portion 2 and a base-end-portion balloon (first expanding member, first balloon) 8 provided closer to the base-end portion than the distal-end portion of the inserted portion 2 is.

The distal-end-portion balloon 7 and the base-end-portion balloon 8 are formed respectively of an elastic material such as polyurethane rubber, silicone rubber, or the like. The distal-end-portion balloon 7 and the base-end-portion balloon 8 are connected to fluid supply pipes (not shown) that supply a fluid for inflating the distal-end-portion balloon 7 and the base-end-portion balloon 8. The fluid supply pipes are disposed inside the inserted portion 2 or along the inserted portion 2.

The distal-end-portion balloon 7 and the base-end-portion balloon 8 are deflated when being inserted into the body, and the inserted portion 2 is accommodated inside the guide sheath 6, with the distal-end-portion balloon 7 and the base-end-portion balloon 8 disposed so as to be aligned with the outer circumferential surface of the inserted portion 2. As shown in Figs. 1A and 1B, in a state in which insertion into the body is completed and the distal-end portion of the inserted portion 2 is made to protrude from the distal-end opening 6a of the guide sheath 6, the distal-end-portion balloon 7 and the base-end-portion balloon 8 are inflated by means for the fluid supplied thereto via the fluid supply pipes.

Here, it is preferable that the distal-end-portion balloon 7 and the base-end-portion balloon 8 block X-rays so that the positions and degrees-of-inflation thereof can easily be ascertained by radiating X-rays from outside the body. In order to block X-rays, for example, an X-ray blocking material, such as lead, tungsten, or the like, is mixed with the elastic material, such as polyurethane rubber, silicone rubber, or the like, with which the distal-end-portion balloon 7 and the base-end-portion balloon 8 are formed, or inner circumferential surfaces of the distal-end-portion balloon 7 and the base-end-portion balloon 8 are coated with an X-ray blocking material. In addition, X-rays can be blocked by using an X-ray imaging agent as the fluid for inflating the distal-end-portion balloon 7 and the base-end-portion balloon 8.

Here, in the inflated state, the distal-end-portion balloon 7 expands toward the outer circumference from the outer circumferential surface 2b of the inserted portion 2. At the same time, the distal-end-portion balloon 7 is inflated so as to overhang further forward than the distal-end surface 2s of the inserted portion 2. As shown in Fig. 2A, it is preferable that the distal-end-portion balloon 7 be such that the outer surface thereof on the opposite side from the side that faces the outer circumferential surface 2b of the inserted portion 2 serves as a convex curved surface 7a that forms a convexity on the opposite side from the inserted portion 2 when viewed from a center C2 of the distal-end-portion balloon 7. By doing so, the contact surface area between the convex curved surface 7a and the parietal pericardium B is increased.

The distal-end-portion balloon 7 is formed only at a portion of the outer circumferential surface 2b of the inserted portion 2 in the circumferential direction, and the outer circumferential surface 2b of the inserted portion 2 is exposed at the remaining portion thereof in the circumferential direction.

As shown in Figs. 1A and 1B, in the direction along the center axis C of the inserted portion 2, the base-end-portion balloon 8 is provided closer to the base-end portion than the distal-end-portion balloon 7 is. To be more specific, it is preferable that the base-end-portion balloon 8 be provided closer to the base-end portion than the bending portion 2a of the inserted portion 2 is so as not to hinder the bending operation of the bending portion 2a. In this case, so long as the bending operation of the bending portion 2a is not hindered, a portion of the base-end-portion balloon 8 may overlap with the bending portion 2a.

As shown in Fig. 2B, it is preferable that the base-end-portion balloon 8 be such that the outer surface thereof on the opposite side from the side that faces the inserted portion 2 serves as a concave curved surface 8a that forms a concavity on the opposite side from the inserted portion 2 when viewed from a center C1 of the base-end-portion balloon 8. By doing so, the contact surface area between the concave curved surface 8a and the surface of the heart A is increased.

As shown in Fig. 3, the base-end-portion balloon 8 may be provided in a ring shape that is continuous over the entire circumference of the inserted portion 2 in the circumferential direction. Alternatively, as shown in Figs. 1A, 1B, and 2B, the base-end-portion balloon 8 may be provided only at a portion of the inserted portion 2 in the circumferential direction, and the outer circumferential surface 2b of the inserted portion 2 may be exposed at the remaining portion in the circumferential direction.

As shown in Fig. 4A, the base-end-portion balloon 8 is disposed on the other side of the distal-end-portion balloon 7, with the inserted portion 2 interposed therebetween, when viewing the cross-section perpendicular to the center axis C of the inserted portion 2. Because of this, when viewing the cross-section perpendicular to the center axis C of the inserted portion 2, at least portions of the base-end-portion balloon 8 and the distal-end-portion balloon 7 respectively exist on a virtual line L that passes through the center axis C of the inserted portion 2 and the observation window 3a of the observation optical system 3. In particular, as shown in Fig. 4B, it is more preferable that the center C1 of the base-end-portion balloon 8 and the center C2 of the distal-end-portion balloon 7 respectively exist on the virtual line L that passes through the center axis C of the inserted portion 2 and the observation window 3a of the observation optical system 3.

For at least one of the distal-end-portion balloon 7 and the base-end-portion balloon 8, it is preferable that width(s) W1 or/and W2 in the direction perpendicular to the virtual line L, which passes through the center axis C of the inserted portion 2 and the observation window 3a of the observation optical system 3, when viewing the cross-section perpendicular to the center axis C of the inserted portion 2, be set to be larger than an outer diameter D of the inserted portion 2. Here, it is preferable that at least the width W2 of the base-end-portion balloon 8 be set to be larger than the outer diameter D of the inserted portion 2.

The operation of the thus-configured endoscope 1A according to this embodiment will be described below.

In order to observe a surface of tissue inside the body, for example, the heart A, by using the endoscope 1A according to this embodiment, the inserted portion 2 is inserted into the pericardial cavity S by passing through the parietal pericardium B in the state in which the inserted portion 2 is accommodated inside the guide sheath 6. In this state, the inserted portion 2 of the endoscope 1A inside the guide sheath 6 is pushed out from the distal-end opening 6a of the guide sheath 6.

Next, the distal-end-portion balloon 7 and the base-end-portion balloon 8 are both inflated.

When the base-end-portion balloon 8 is inflated, the inserted portion 2 is moved away from the surface of the heart A. By doing so, a sufficient distance is ensured between the observation window 3a provided in the distal-end surface 2s of the inserted portion 2 and the surface of the heart A, and thus, an appropriate observation distance is formed therebetween. When the distal-end-portion balloon 7 is inflated, the distal-end-portion balloon 7 extends forward from the distal-end portion of the inserted portion 2 similarly to eaves. At the same time, the distal-end-portion balloon 7 supports the parietal pericardium B so as to prevent the parietal pericardium B from sagging. At this point, as shown in Fig. 1B, by bending the bending portion 2a and by acquiring an image of the surface of the heart A by operating the observation optical system 3, it is possible to check the affected portion of the surface of the heart A.

Once the procedure is completed, the distal-end-portion balloon 7 and the base-end-portion balloon 8 are deflated by using the manipulating portion close at hand, and the inserted portion 2 and the guide sheath 6 are removed.

As has been described above, by inflating the distal-end-portion balloon 7 and the base-end-portion balloon 8, respectively, because it is possible to ensure a sufficient gap and space between the distal-end portion of the inserted portion 2 and the surface of the heart A, it is possible to prevent narrowing of the observation viewing field of the observation optical system 3 and it is also possible to ensure a sufficient space for performing treatment.

When inserting the inserted portion 2 into the pericardial cavity S, the distal-end-portion balloon 7 and the base-end-portion balloon 8 are used to block X-rays, and thus, it is possible to easily ascertain the positions and the degrees-of-inflation of the distal-end-portion balloon 7 and the base-end-portion balloon 8 inside the body by radiating X-rays from outside the body.

By configuring the outer surface of the distal-end-portion balloon 7 so as to serve as the convex curved surface 7a that forms a convexity on the opposite side from the inserted portion 2 when viewed from the center C2 of the distal-end-portion balloon 7, the distal-end-portion balloon 7 conforms to the parietal pericardium B, and thus, it becomes easier to stabilize the orientation thereof with respect to the parietal pericardium B. Furthermore, if the width W1 of the distal-end-portion balloon 7 is set to be larger than the outer diameter D of the inserted portion 2, it is possible to support the parietal pericardium B across a greater width, and it is possible to more reliably ensure a sufficient viewing field in the observation optical system 3.

By configuring the outer surface of the base-end-portion balloon 8 so as to serve as the concave curved surface 8a that forms a concavity on the opposite side from the inserted portion 2 when viewed from the center C1 of the base-end-portion balloon 8, the base-end-portion balloon 8 conforms to the surface of the heart A, and thus, it is possible to stably support the inserted portion 2 by preventing the inserted portion 2 from rotating about the center axis C. Furthermore, by setting the width W2 of the base-end-portion balloon 8 to be larger than the outer diameter D of the inserted portion 2, the inserted portion 2 is even more stably supported.

With these configurations, the observation viewing field of the observation optical system 3 is more reliably ensured.

The distal-end-portion balloon 7 and the base-end-portion balloon 8 are disposed on the both sides of the inserted portion 2 when viewing the cross-section perpendicular to the center axis C of the inserted portion 2. By doing so, it is possible to maximize the distance between the heart A and the parietal pericardium B, and it is possible to ensure a maximally large viewing field in the observation optical system 3 at the distal-end portion of the inserted portion 2.

As shown in Fig. 3, by providing the base-end-portion balloon 8 only at a portion of the inserted portion 2 in the circumferential direction, as compared with the case in which the base-end-portion balloon 8 is provided so as to be continuous over the entire circumference of the inserted portion 2 in the circumferential direction, it is possible to move the inserted portion 2 further away from the heart A. Accordingly, the viewing field of the observation optical system 3 can be made larger.

As has been described above, with the endoscope 1A according to this embodiment, it is possible to prevent narrowing of the observation viewing field and it is also possible to ensure a sufficient space for performing treatment.

Because the endoscope 1A is inserted and removed in the state in which the distal-end-portion balloon 7 and the base-end-portion balloon 8 are deflated by using the manipulating portion close at hand, it is possible to decrease the burden on the patient.

Next, a plurality of other embodiments of the present invention will be described. In the other embodiments described below, configurations that differ from the configurations of the above-described first embodiment will mainly be described, and configurations common with those of the above-described first embodiment will be given the same reference signs, and descriptions thereof will be omitted.

### {Second Embodiment}

As shown in Fig. 5, an endoscope 1B according to a second embodiment of the present invention is provided with the inserted portion 2, the observation optical system 3, and a supporting expansion portion 4B.

The supporting expansion portion 4B in this embodiment is provided with the distal-end-portion balloon 7 provided at the external side surface of the distal-end portion of the inserted portion 2, the base-end-portion balloon 8 provided closer to the base-end portion than the distal-end portion of the inserted portion 2 is, and a bending balloon (third balloon) 10 that is provided closer to the base-end portion of the inserted portion 2 than the base-end-portion balloon 8 is and that bends the inserted portion 2.

The bending balloon 10 is disposed on the opposite side of the base-end-portion balloon 8 with the inserted portion 2 interposed therebetween. It is preferable that the bending balloon 10 and the distal-end-portion balloon 7 be coaxially positioned at the outer circumferential surface 2b of the inserted portion 2.

Here, the bending balloon 10 is provided closer to the base-end portion of the inserted portion 2 than the base-end-portion balloon 8 is. In more detail, in Figs. 1A and 1B, in the state in which the base-end-portion balloon 8 is inflated and the inserted portion 2 is moved away from the surface of the heart A, it is preferable that the bending balloon 10 be provided, as shown in Fig. 5, between a portion P1 at which the base-end-portion balloon 8 is in contact with the inserted portion 2 and a portion P2 that is closer to the base end of the inserted portion 2 than the portion P1 is and at which the inserted portion 2 is in contact with or in the close vicinity of the surface of the heart A.

It is preferable that the bending balloon 10 be such that the outer surface thereof on the opposite side from the side that faces the inserted portion 2 serves as a convex curved surface 10a that forms a convexity on the opposite side from the inserted portion 2 when viewed from a center C3 of the bending balloon 10.

Here, as shown in Fig. 6, the bending balloon 10 may be provided in a ring shape that is continuous over the entire circumference of the inserted portion 2 in the circumferential direction. Alternatively, as shown in Fig. 5, the bending balloon 10 may be provided only at a portion of the inserted portion 2 in the circumferential direction, and the outer circumferential surface 2b of the inserted portion 2 may be exposed at the remaining portion in the circumferential direction.

As shown in Fig. 7, it is preferable that a width W3 of the bending balloon 10 also be set to be larger than the outer diameter D of the inserted portion 2. By doing so, because surface areas of the bending balloon 10 that are in contact with the parietal pericardium B and the heart A are increased, it is possible to prevent the inserted portion 2 from rotating about the center axis C and to stably support the inserted portion 2.

The operation of the thus-configured endoscope 1B according to this embodiment will be described below.

In order to observe a surface of tissue inside the body, for example, the heart A, by using the endoscope 1B according to this embodiment, the inserted portion 2 is inserted into the pericardial cavity S by passing through the parietal pericardium B in the state in which the inserted portion 2 is accommodated inside the guide sheath 6. In this state, the inserted portion 2 of the endoscope 1B in the guide sheath 6 is pushed out from the distal-end opening 6a of the guide sheath 6.

Next, the individual balloons 7, 8, and 10 individually are inflated.

When the base-end-portion balloon 8 is inflated, the inserted portion 2 is moved away from the surface of the heart A. By doing so, a sufficient distance is ensured between the observation window 3a provided in the distal-end surface 2s of the inserted portion 2 and the surface of the heart A, and thus, an appropriate observation distance is formed therebetween. Then, when the bending balloon 10 is inflated, the outer surface of the bending balloon 10 comes into contact with the parietal pericardium B, and the bending balloon 10 presses the inserted portion 2 toward the heart A by receiving a reaction force from the parietal pericardium B. The bending balloon 10 presses the inserted portion 2 toward the heart A between the portion P1 at which the base-end-portion balloon 8 is in contact with the inserted portion 2 and the portion P2 that is closer to the base end of the inserted portion 2 than the portion P1 is and at which the inserted portion 2 is in contact with the surface of the heart A. As a result, the inserted portion 2 is bent and is raised in the direction that moves the inserted portion 2 away from the outer circumferential surface of the heart from a position aligned with the heart A, after which the distal-end portion is aligned with the base-end-portion balloon 8, thus bending the inserted portion 2 in an S-shape. By doing so, the distal-end portion of the inserted portion 2 is made nearly parallel to the surface of the heart A and the parietal pericardium B.

When the distal-end-portion balloon 7 provided at the distal-end portion of the inserted portion 2 is inflated, the distal-end-portion balloon 7 extends forward from the distal-end portion of the inserted portion 2 similarly to eaves. At the same time, the distal-end-portion balloon 7 supports the parietal pericardium B and prevents the parietal pericardium B from sagging.

At this point, by acquiring an image of the surface of the heart A by operating the observation optical system 3, it is possible to check the affected portion of the surface of the heart A.

Once the procedure is completed, the distal-end-portion balloon 7 and the base-end-portion balloon 8 are deflated by using the manipulating portion close at hand, and the inserted portion 2 and the guide sheath 6 are removed.

With the endoscope 1B according to this embodiment described above, as with the first embodiment described above, it is possible to prevent narrowing of the observation viewing field and it is also possible to ensure a sufficient space for performing treatment.

As compared with the endoscope 1A, with the endoscope 1B that has the bending balloon 10, the restoring force of the bent inserted portion 2 acts with a greater force and works against the force that acts to bring the parietal pericardium B closer to the heart A. By bending the inserted portion 2 by using the bending balloon 10, the distal-end portion of the inserted portion 2 can be made nearly parallel to the surface of the heart A and the parietal pericardium B. By doing so, it is possible to perform observation and treatment by making the inserted portion 2 parallel to the surface of the heart A. When the surface of the heart A and the inserted portion 2 are parallel to each other, the space in front of the distal-end surface 2s is increased in the state in which a sufficient distance to the heart A is ensured.

Note that the configuration described in the second embodiment described above can be modified as below.

For example, the base-end-portion balloon 8 and the bending balloon 10 may be formed as a single unit.

Without limitation to the heart, the endoscopes 1A and 1B can similarly be disposed on other organs.

When viewing the cross-section perpendicular to the center axis C of the inserted portion 2, at least portions of the individual balloons 7, 8, and 10 may respectively exist on the virtual line L that passes through the center axis C of the inserted portion 2 and the observation window 3a of the observation optical system 3. In particular, it is more preferable that centers of the individual balloons 7, 8, and 10 respectively exist on the virtual line L that passes through the center axis C of the inserted portion 2 and the observation window 3a of the observation optical system 3.

In the endoscopes 1A and 1B according to the individual embodiments described above, although the endoscope 1A is disposed on the heart A, it is not limited thereto. For example, the endoscope 1A may be disposed below the heart A in a state in which the base-end-portion balloon 8 is disposed on the heart A side and the distal-end-portion balloon 7 is disposed on the parietal pericardium B side.

In addition, one of each of the individual balloons 7, 8, and 10 may be provided, or more than one of each may be provided.

### {Reference Signs List}

1A, 1B endoscope
2 inserted portion
2a bending portion
2b outer circumferential surface
2s distal-end surface
3 observation optical system
3a observation window
4A supporting expansion portion
4B supporting expansion portion
6 guide sheath
7 distal-end-portion balloon (second expanding member, second balloon)
7a convex curved surface
8 base-end-portion balloon (first expanding member, first balloon)
8a concave curved surface
10 bending balloon (third balloon)
10a convex curved surface

## Claims

1. An endoscope comprising:
an inserted portion that is inserted into a body;
an observation optical system that is provided at a distal-end portion of the inserted portion and that acquires an image of the body interior; and
a supporting expansion portion that makes an organ inside the body support the distal-end portion of the inserted portion and that is provided with a first expanding member, which is provided closer to a base-end portion than the distal-end portion of the inserted portion is, and a second expanding member, which is provided so as to overhang forward from the distal-end portion of the inserted portion,
wherein, when viewing a cross-section perpendicular to a center axis of the inserted portion, the first expanding member is disposed on the opposite side from the second expanding member, with the inserted portion interposed therebetween.

2. An endoscope according to Claim 1,
wherein the first expanding member is a first balloon that is inflated toward an outer circumferential side from the inserted portion, and
the second expanding member is a second balloon that is inflated toward the outer circumferential side from the inserted portion and that is inflated so as to overhang further forward than the distal-end portion of the inserted portion.

3. An endoscope according to Claim 2,
wherein the supporting expansion portion is additionally provided with a third balloon provided closer to the base-end portion of the inserted portion than the first balloon is and that bends the inserted portion, and
the third balloon is disposed on the opposite side from the first balloon, with the inserted portion interposed therebetween.

4. An endoscope according to Claim 3, wherein, in a state in which the first balloon is inflated and the inserted portion is moved away from a surface of the organ, the third balloon is provided between a portion at which the first balloon is in contact with the inserted portion and a portion at which the inserted portion is in contact with the surface of the organ.

5. An endoscope according to Claim 2, 3, or 4, wherein the supporting expansion portion is formed by being provided with a material that blocks an X-ray.

6. An endoscope according to Claim 2, 3, or 4, wherein an outer surface of the first balloon that is on an opposite side from a side that faces the inserted portion serves as a concave curved surface that forms a concavity on an opposite side from the inserted portion when viewed from a center of the first balloon.

7. An endoscope according to Claim 2, 3, or 4, wherein an outer surface of the second balloon on an opposite side from a side that faces an outer circumferential surface of the inserted portion serves as a convex curved surface that forms a convexity on an opposite side from the inserted portion when viewed from a center of the second balloon.

8. An endoscope according to Claim 2, 3, or 4, wherein the first balloon is provided only at a portion of an outer circumferential surface of the inserted portion in a circumferential direction, and the outer circumferential surface of the inserted portion is exposed at a remaining portion in the circumferential direction.

9. An endoscope according to Claim 2, 3, or 4, wherein the first balloon is provided closer to the base-end portion than a bending portion of the inserted portion is.

10. An endoscope according to Claim 2, 3, or 4, wherein at least one of the first balloon and the second balloon has, at a cross-section perpendicular to the center axis of the inserted portion, a width set to be larger than an outer diameter of the inserted portion.

11. An endoscope according to Claim 2, 3, or 4, wherein at least the width of the first balloon is set to be larger than an outer diameter of the inserted portion.

12. An endoscope according to Claim 3 or 4, wherein an outer surface of the third balloon on an opposite side from a side that faces the inserted portion serves as a convex curved surface that forms a convexity on an opposite side from the inserted portion when viewed from a center of the third balloon.

13. An endoscope according to Claim 3, or 4, wherein the third balloon is provided only at a portion of the inserted portion in a circumferential direction, and the outer circumferential surface of the inserted portion is exposed at a remaining portion in the circumferential direction.

14. An endoscope according to Claim 12 or 13, wherein at least one of the first balloon, the second balloon, and the third balloon has, at a cross-section perpendicular to the center axis of the inserted portion, a width set to be larger than an outer diameter of the inserted portion.
